# EUROPEAN PATENT SPECIFICATION

(11) **EP 2 748 578 B1**
(45) Date of publication and mention of the grant of the patent: **31.07.2019**
(21) Application number: 12748509.2
(22) Date of filing: 22.08.2012
(51) Int. Cl.: G01N 1/20, G01N 33/02

(54) **SAMPLING DEVICE FOR FREE FLOWING PARTICULATE MATERIAL**
PROBENNAHMEVORRICHTUNG FÜR RIESELFÄHIGES SCHÜTTGUT
DISPOSITIF D'ÉCHANTILLONNAGE POUR MATÉRIAU PARTICULAIRE S'ÉCOULANT LIBREMENT

(30) Priority: 22.08.2011 SE 1100612
(43) Date of publication of application: 02.07.2014
(73) Proprietor: Tornum AB, 535 22 Kvänum (SE)
(72) Inventor: ANDERSSON, Rolf, S-534 94 Vara (SE)
(74) Representative: Valea AB
(86) International application number: PCT/EP2012/066351
(87) International publication number: WO 2013/026881

(56) References cited:
- EP-A2- 0 034 459
- WO-A1-89/10548
- FR-A1- 2 342 917
- FR-A1- 2 547 638

## Description

### TECHNICAL FIELD

The present invention relates to a sampling device for the sampling and analysis of free flowing particulate material, such as cereal grains. The invention also relates to a method for using the sampling device, as well as calibration of the same.

### BACKGROUND OF THE INVENTION

Cereal grains and oil plants are to a greater extent dried and stored at the farms waiting to be sold at a better price or delivery by contract. The costs for the handling of cereal grains may be lowered considerably if it can be transported directly from the primary source to the processing party without having to pass by a commercial storage. This assumes that its quality may be assessed before sale, based on sampling directly at the farm. Knowledge about the quality of the cereal is also important during the preparation of animal feed. Important parameters for assessing the quality of a lot are e.g. the level of molds and mycotoxins, protein, fall number, temperature, density, water activity and moisture content. A correct sampling is a prerequisite for acceptable accuracy in connection with quality analysis. A prerequisite for a representative sampling is that all particles in the Iot to be sampled have the same possibility to end up in the sample. Regarding cereals there are two types of samplers, those which sample the grains when they are in motion by sampling the complete cross section of the flow at an even time interval. This will occur automatically (mechanical sample diverter, for example from a waste pipe) or manually by means of a special designed shovel. Sampling may also take place by means of a sampling poke which will sample cereal at rest in a load or shallow layers, Sampling of a flow is considered to be more reliable as the grains in general are sampled more frequently as well as providing a more representative sample.

A great part of the cereal harvested has a moisture content that is too high to be stored right away. To assure a good storage capabilities for the dried cereal and at the same time prevent costly over-drying; a good supervision of the moisture content is essential. If the water measuring device shows a reading for the moisture content that is too low, it means that the cereal lot contains more moisture than it ought to and further drying may be required before delivery. A moisture content that is too high may also deteriorate the quality during storage. If the measuring device instead shows a moisture content that is too high there is a risk that the grains are dried too much, which will cost.

When measuring the water content there are two different methods: direct and indirect.

The direct method means that the water is removed by drying. The sample is weighed, maybe milled, dried and weighed again. The method is reliable and is insensitive to the special characteristics of the crop. A drawback with this method is that the sample preparation (milling) and drying of the sample is time consuming.

The indirect method measures the electrical characteristics of the cereal. A drawback with the method is that it measures a characteristic that is only partly dependent on the moisture content. The great advantage with this type of measuring device is that the handling is easy and the result is obtained quickly. To have an exact measuring device as possible may save time and money. There are presently a few so called fast measuring devices on the market which will measure the moisture content in a few seconds, i.e. often using an indirect method. However, it is not unusual that many of these measuring devices deviate from a reference meter. The error in measurement in these fast measuring devices for moisture content may be up to 1.5 percent. Calibration of fast measuring devices may often only be made by the supplier and in order for the error of measurement not to increase the calibration should be repeated every year.

The document WO8910548 A1 discloses a device and a process for on-line measurement of product parameters, wherein a subset of said product is diverted into a measuring chamber.

The document FR2547638 A1 relates to an apparatus for selecting directions for granular or powdered products.

The present invention provides a sampling equipment wherein the advantage of the handling of the fast measuring device is combined with the possibility of calibrating the measuring device quickly and easily as often as you like without having to send the measuring device off to the supplier in order to assure that the device shows a correct result.

### SUMMARY OF THE INVENTION

The present invention relates to a sampling device, according to claim 1, for the sampling and analysis of free flowing particulate material in a moving flow of said material.

The sample diversion device may further comprise a sample diversion channel with a breaking device for distributing the flow of the free flowing particulate material through the sampling diversion channel into the measuring container.

The measuring device may be designed to measure parameters from the group consisting of protein content, moisture content, fall number, temperature, density, water activity and the presence of molds and mycotoxins in the free flowing particulate material.

The measuring device may be designed to measure moisture content in the free flowing particulate material.

The measuring device may be designed to measure protein content in the free flowing particulate material.

The sample discharging device is slidably arranged between
(I) a closed position: wherein the outlet opening of the measuring container is closed; or
(II) a discharging position: wherein the discharging channel cooperates with the measuring container; or
(III) a calibration position: wherein the calibration channel cooperates with the measuring container.

The calibration channel has a volume X which equals the volume X of the measuring container.

The calibration channel has an outlet opening that may be arranged to cooperate with a collection container.

The free flowing particulate material may be of the group consisting of kernels and seeds from leguminous plants cruciferous plants, cereals grass and rice.

The free flowing particulate material may be of the group consisting of barley, wheat, corn, rye, brown rice, oats, rape seeds, sunflower seeds, malt, durra, peas, soya beans, peanuts, shelled peanuts, corn kernels, cotton seed, white rice and/or grass.

The free flowing particulate material may be kernels or seeds from cereals.

The invention further comprises a method, according to claim 10, for sampling and analysis of samples from a moving flow of free flowing particulate material, by means of the sampling device mentioned above, as well as calibration of said sampling device, wherein said method comprises the following steps:
a) a subset of free flowing particulate material is diverted by means of the sampling device from the moving flow of free flowing particulate material to the measuring container having the volume X; and
b) the sample discharging device is slidably arranged to a closed position (I), whereby the measuring container is filled with a sample Y of free flowing particulate material having the volume X, and the measuring device records one or more of the chosen sample parameters in said sample Y; and
c) the sample discharge device is slidably arranged either
   (c-1) to the discharge position (II): whereby said sample Y having the volume X is moved from the measuring container to the discharge channel and is discharged from the sampling device; or
   (c-2) to the calibration position (III): whereby said sample Y having the volume X is transferred from the measuring container to the calibration channel, and thereafter to a collection container for external assessment of said one or more chosen sample parameter; whereafter
(d) (a) - (d) is repeated.

In (a) the subset of free flowing particulate material may continuously be diverted from the moving flow of free flowing particulate material.

In (b) a breaking device may distribute the free flowing particulate material in the measuring container.

In (b) the measuring device may record one or more chosen sample parameters from the group consisting of protein content, moisture content, fall number, temperature, density, water activity and/or the presence of molds and mycotoxins in said sample Y having the volume X.

In (c-1) said sample Y having the volume X is transferred from the measuring container to the discharge channel) and is restored to the moving flow of free flowing particulate material.

In (c-2) after said sample Y having the volume X has been transferred to the collection container, the method may further comprise the following steps:
(i) the same one or more chosen sample parameters recorded in (b), are assessed by means of an external measuring device in said sample Y having the volume X, and
(ii) the value of the one or more chosen sample parameters obtained by means of the external measuring device in (i) is compared to the value of the one or more chosen sample parameters obtained by means of the measuring device in (b); and
(iii) if the values for the one or more of the chosen sample parameters obtained by means of the external measuring device in (i) and the measuring device in (b) respectively are the same or close, (a), (b), (c-1) and (d) are repeated, but if the values are not the same or close the measuring device is readjusted and (a), (b), (c-2) and (d) are repeated.

### BRIEF DESCRIPTION OF THE DRAWINGS

Figure 1 shows the sampling device according to the invention
Figure 2 shows the sampling device in closed position (I) wherein the outlet of the measuring container is closed and the measurement of one or more chosen parameters takes place.
Figure 3 shows the sampling device in a discharging position (II) wherein the measuring container cooperates with the discharging container and discharge of the sample takes place.
Figure 4 shows the sampling device in a calibration position (III) wherein the measuring container cooperates with the calibration container and the sample is transferred from the measuring container to the calibration container.
Figure 5 shows the sampling device when the sample Y having the volume X is transferred to the collection container.

### DETAILED DESCRIPTION OF THE INVENTION

With reference to the figures in which similar elements have identical reference numerals the sampling device 1 according to the invention will now be described in more detail.

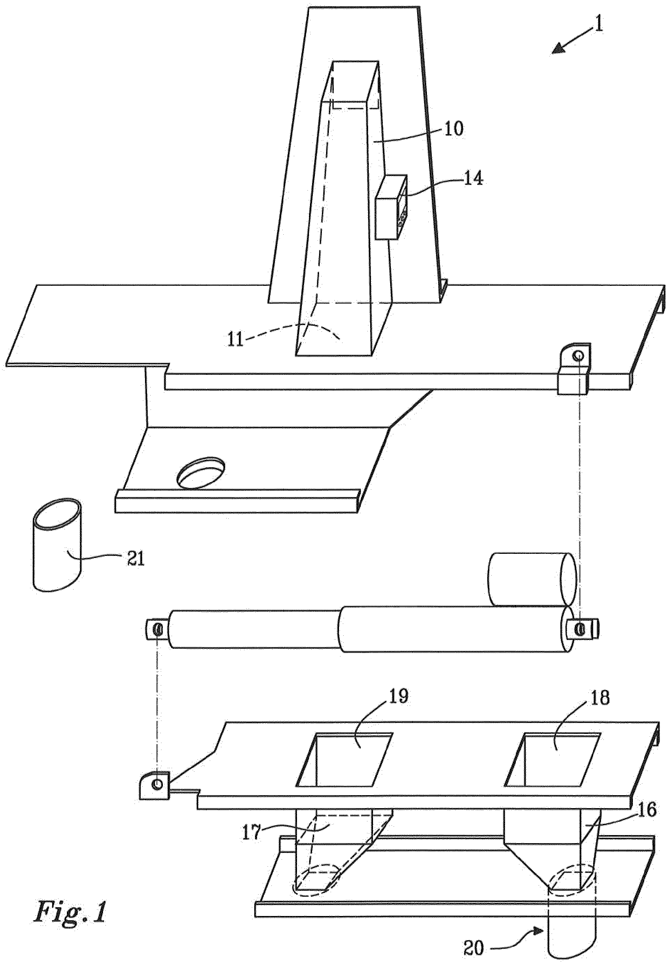

Figure 1 shows the different parts of the sampling device 1. The sampling device 1 is generally intended to be used in sampling and analysis of free flowing particulate material 2 from a moving flow 3 of said material. Examples of free flowing particular material 2 may be kernels and seeds from leguminous plants cruciferous plants, grass rice and especially from cereals. Specific examples of free flowing particulate material 2 may be kernals and seeds of the group consisting of barley, wheat, corn, rye, brown rice, oats, rape seeds, sunflower seeds, malt, durra, peas, soya beans, peanuts, shelled peanuts, corn kernels, cotton seed, white rice and/or grass.

A condition for correct sampling is that all particles in the sampled lot have the same opportunity to end up in the sample. Regarding free flowing particulate material, and especially regarding cereals it is an advantage if the sampling procedure is accomplished when the free flowing particulate material is transferred in a moving flow. Thus, the sampling device 1 according to the invention is advantageously arranged in connection with a chute or shoot 4, wherein the free flowing particulate material 2 is transferred in a moving flow 3.

The chute 4 may be part of a dryer system, transporting device or storage container for free flowing particulate material 2. The sampling device 1 further comprises a sample diversion device 5 for diverting a subset of the free flowing particulate material 2 from the moving flow 3 of free flowing particulate material.

The sample diversion device 5 has the shape of a tube or funnel and is arranged through an opening 6 in the wall 7 of the chute 4. The inlet opening 8 of the sampling diversion device is located in the moving flow 3 of the free flowing particulate material in order for a subset of the free flowing particulate material 2 to be diverted into the inlet opening 8 of the sampling diversion device. Advantageously the sample diversion device 5 is arranged and designed to withdraw (or catch) a sample that is representative for the complete lot of the free flowing particulate material 2. The sample diversion device 5 may optionally be provided with a closing mechanism to prevent the flow of free flowing particulate material from entering the sampling device 5 (not shown).

The diverted sample is thereafter directed from the sample diversion device 5 via a sample diversion channel 9 into a measuring container 10 which is advantageously arranged on the outside of the wall 7 of the chute.

The measuring container 10 comprises a certain volume X that may vary between sampling devices, and has an outlet opening 11 in the bottom part.

Free flowing particulate material 2 of the type that has been described above is comprised in the group of heterogenic particulate material which may result in a tendency to form layers during movement, i.e. a physical separation may occur. A layering of the sample in the measuring container 10 may influence the obtained result in a negative way and consequently the sample diversion channel 9 according to the present invention advantageously comprises a breaking device 12 to distribute the moving flow 3 of free flowing particulate material 2 through the sample diversion channel 9 down into the measuring container 10. The breaking device 12 is constituted by a lip or protrusion arranged on the inside of the wall of the sample diversion channel 9, onto which each individual particle hits before they are distributed in the measuring container 10.

The sampling device 1 further comprises a measuring device 14 advantageously arranged in connection to the measuring container 10. Said measuring device is designed to record one or more chosen parameters for the free flowing particulate material 2. The chosen parameters include, but are not limited to protein content, moisture content, fall number, temperature, density, water activity and/or the presence of molds and mycotoxins in the free flowing particulate material 2, but also the recording of other parameters known to the person skilled in the art are conceivable.

The sampling device 1 further comprises a sample discharging device 15 having a discharging channel 16 and a calibration channel 17, said sample discharging device 15 is slidably arranged to cooperate with the outlet opening 11 of the measuring container 10. The discharging channel 16 as well as the calibration channel 17 each comprises an inlet opening 18 and 19 respectively. The discharging channel 16 also comprises an outlet opening that ends up into a discharging tube 20, while the outlet of the calibration channel is advantageously arranged to cooperate with a collection container 21 for the sample to be calibrated. The calibration channel 17 has a volume X which equals the volume X of the measuring container 10. The importance of this feature will be described below.

Advantageously the sample discharging device is arranged to let the free flowing particulate material 2 located in the measuring container to transfer itself due to gravity into the discharging channel 16 or calibration channel 17 respectively. Advantageously the sample discharging device 15 is arranged underneath the outlet opening 11 of the measuring container.

The sample discharging container 15 is slidably arranged between the following positions: the closed position (I): wherein the outlet opening 11 of the measuring container is closed; or
the discharging position (II): wherein the discharging channel 16 cooperates with the measuring container 10; or
the calibration position (III): wherein the calibration channel 17 cooperates with the measuring container 10.

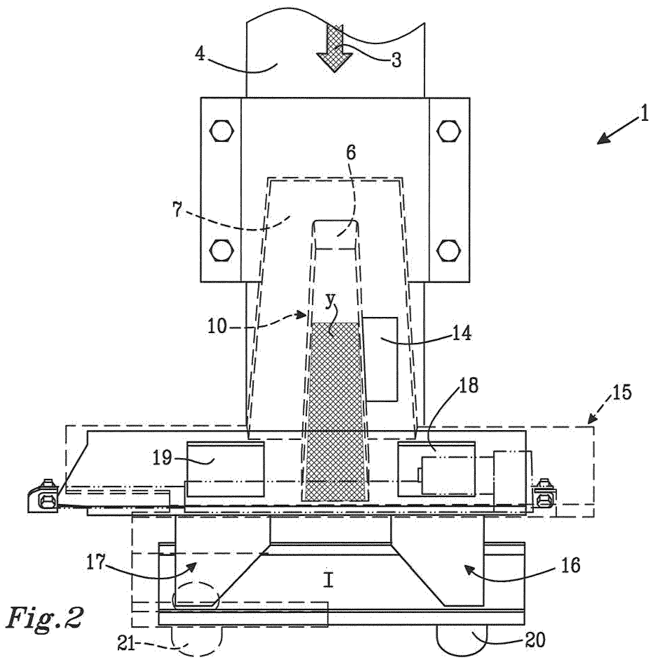

In the closed position (I) the sample discharging device 15 is arranged to a position wherein the outlet opening 11 of the measuring container is closed. This is achieved by sliding the sample discharging device 15 to a position adjacent the inlet openings of the discharging channel 18 and calibration channel 19 respectively. The sampling device 1 shown in figure 2 is in the closed position between the inlet openings of the discharging channel 16 and the calibration channel 17. When in this position the free flowing particulate material 2 will remain in the measuring container 10.

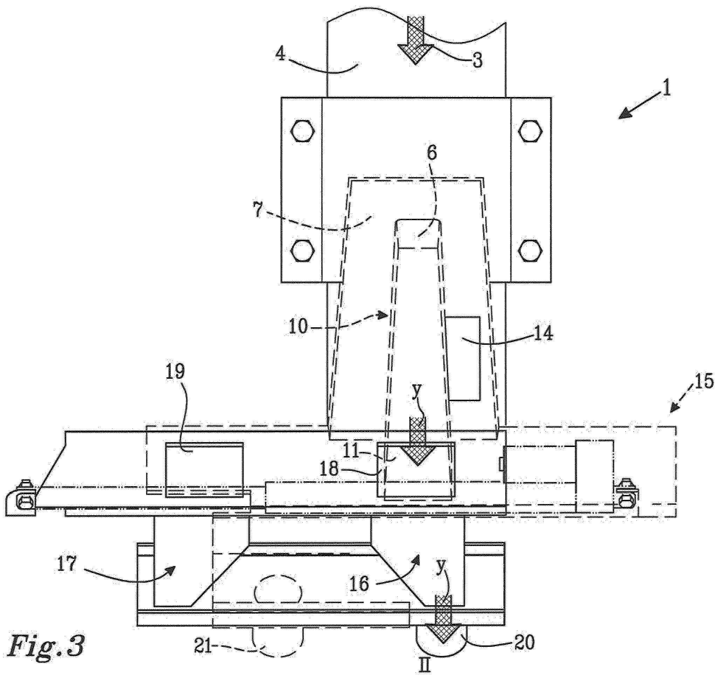

In the discharging position (II) the sample discharging device 15 is arranged in a position wherein the inlet opening 18 of the discharging channel substantially overlaps and covers the outlet opening 11 of the measuring container. In this position the volume X of free flowing particulate material 2 present in the measuring container 10 is transferred to the discharging channel 16, advantageously by means of gravity (see figure 3).

The discharging container 16 further comprises a discharging tube 20 through which the free flowing particulate material 2 present in the discharging container 16 may be discharged or be returned to the moving flow 3 of free flowing particulate material 2.

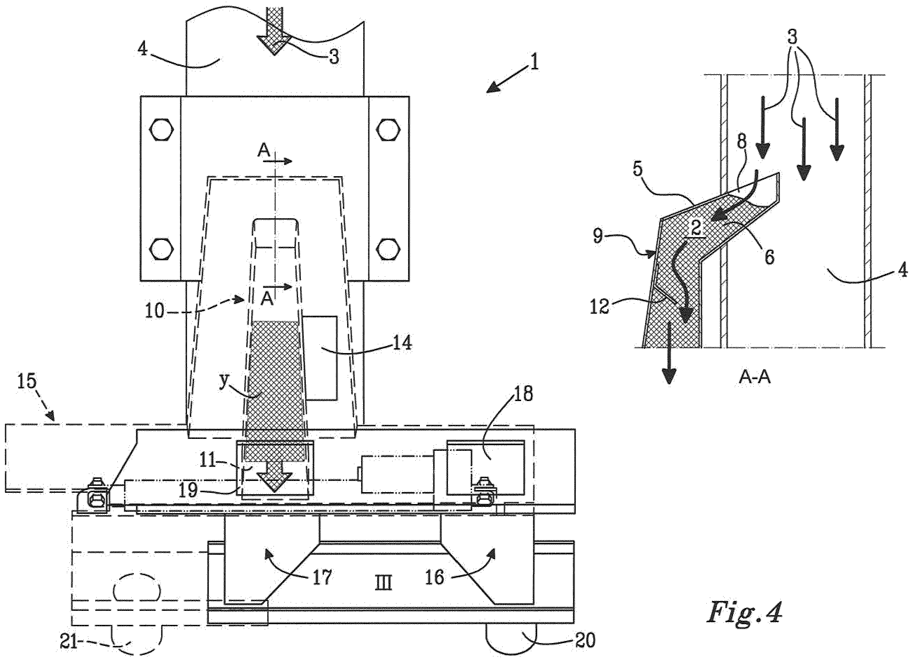

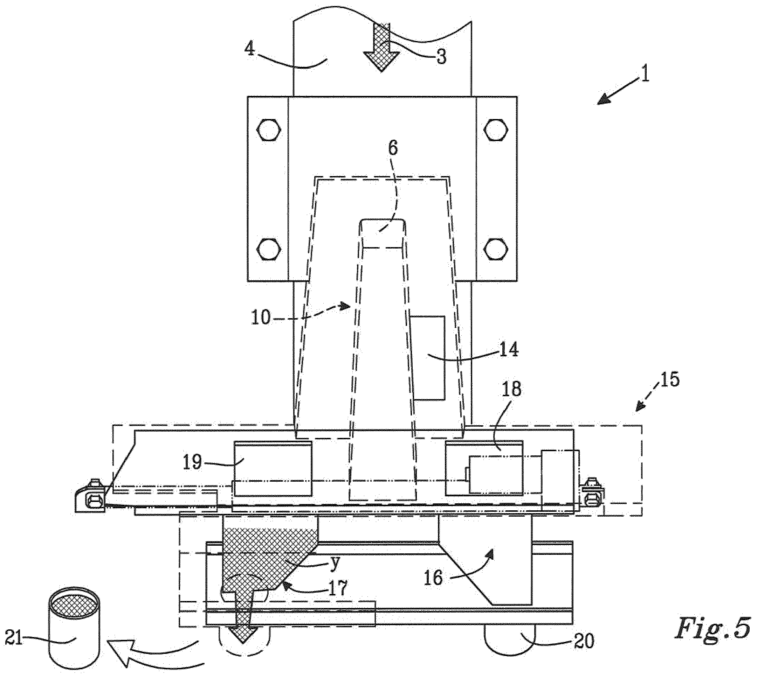

In the calibration position (III) the sample discharging device 15 is arranged in a position wherein the inlet opening 19 of the calibration channel is substantially overlapping and covering the outlet opening 11 of the measuring container. In this position the volume X of free flowing particulate material 2 present in the measuring container 10 is transferred to the calibration channel 17, advantageously by means of gravitation (see figure 4). A collection container 21 for the calibration sample may optionally be connected to the outlet opening of the calibration channel, from where the sample may be removed (see figure 5) and the chosen parameter may be recorded by means of an external measuring device (not shown).

Sliding of the sample discharging device 15 between the different positions I-III may be accomplished in many ways. Possible solutions would be sliding by means of hydraulics, pneumatics, or electronics which may be maneuvered manually or by a computer. The person skilled in the art realizes that the manner by which the sliding of the sample discharging device 15 is accomplished is not limiting to the invention.

A method for sampling and analysis of a sample from a moving flow 3 of free flowing particulate material 2, by means of the sampling device 1 of the invention, as well as the calibration of said sampling device 1 will now be described in more detail, wherein said method comprises the following steps:
a) a subset free flowing particulate material 2 is diverted by means of the sample diversion device 5. Said device is advantageously arranged in connection with a chute or shoot 4 wherein the free flowing particular material 2 flows. The diverted sample is thereafter directed from the sample diversion device 5 via a sample diversion channel 9 to the measuring container 10 having the volume X. Said measuring container is advantageously arranged on the outside of the wall 7 of the chute 4. The free flowing particulate material may continuously be diverted to the sample diversion device 5 or collected manually at certain intervals when the sample diversion device 5 opens up momentarily.
b) during collection of a sample the sample discharging device 15 is slidably arranged to a closed position (I), whereby the measuring container 10 is filled with a sample of free flowing particulate material 2 having the volume X. In order to prevent layering of the diverted sample in the measuring container 10, which may influence the obtained result in a negative way, the sample diversion channel 9 according to the invention is provided with a breaking device 12, which will break the moving flow 3 of free flowing particulate material 2 through the sample diversion channel 9. Consequently the sample Y having the volume X is distributed without forming layers in the measuring container 10. When the measuring container 10 has been filled with the sample Y having the volume X the measuring device 14 will record one or more chosen sample parameters from the group consisting of protein content, moisture content, fall number, temperature, density, water activity and/or the presence of molds and mycotoxins in the free flowing particulate material 2, but also other parameters known to the person skilled in the art may be assessed by the measuring device of the present invention.
c) when the measuring container has been filled with sample Y having the volume X and one or more of the chosen sample parameters have been assessed, the sample discharge device 15 is slideably arranged either
   c-1) to the discharging position (II), whereby said sample Y having the volume X is transferred to the discharging channel 16, and said sample Y having the volume X is returned to the moving flow of free 3 flowing particulate material 2 through the discharging tube 20; or alternatively the sample discharging device 15 is slideably arranged to
   c-2) the calibration position (III), whereby said sample Y having the volume X is transferred to the calibration channel 17, and thereafter to a collection container for the calibration sample 21. Withdrawal of the calibration sample may be arranged at periodically pre-programmed occasions controlled by an external or internal operating system, or manually according to need.

Thereafter the same one or more chosen parameters measured in step b for sample Y having the volume X, are measured again, but this time using an external measuring devise. The external measuring device may be the same equipment that is normally used for calibrating the fast equipment, or equipment that measures the same parameters as the chosen parameter but by using some other method for analysis. The person skilled in the art is familiar with the type of measuring equipment that may be used when comparing the chosen sample parameters. Subsequently the results for the one or more chosen parameters obtained by the external measuring equipment are compared with the result recorded by the measuring device 14 in step b. If the results for the one or more chosen sample parameters obtained by the external measuring equipment and the measuring device 14 respectively, are the same or similar, the measuring device is considered to function satisfactory, and no adjustment of the measuring device is required. The steps a), b), c-1 are repeated, i.e. a) the measuring container 10 is refilled with a sample Y having the volume X, b) the measuring container 10 is closed and one or more sample parameters are recorded, and c-1) the sample is dischargeed, until the next withdrawal of a calibration sample (i.e. step c-2) is started.

If the sample parameters obtained by the external measuring device are different from the results obtained by the measuring device 14 in b), the measuring device 14 is adjusted, and the steps a), b) and c-2) are repeated. This means that step a) the filling of the measuring container with sample Y having the volume X, b) closing the measuring container 10 och recording of one or more sample parameters and, c-2 withdrawal of a calibration sample. The obtained result from the calibration sample is compared as described above, and is repeated until the results for the one or more chosen parameters obtained by the external measuring equipment and the measuring device 14 in b) respectively are the same or similar. Thereafter the steps a), b), c-1, are repeated, i.e. a) re-filling of sample Y having the volume X in the measuring container 10, b) closing of the measuring container 10 och recording of one or more sample parameters and, c-1 sample discharge, until next withdrawal of calibration sample (c-2) is initiated.

It should be emphasized that the sample Y having the volume X analysed by the external measuring equipment must be the same as was examined by the measuring device 14 in step b. This is to avoid any measurement errors that may occur if the measurements are made on different samples.

## Claims

1. A sampling device (1) for sampling and analysis of free flowing particulate material (2) in a moving flow (3) of said material, comprising a sample diversion device (5) to divert part of the free flowing particulate material (2) from the moving flow (3) of free flowing particulate material to a measuring container (10) having a volume X, and a measuring device (14) arranged in connection to the measuring container (10) to record a chosen sample parameter for the free flowing particulate material (2); wherein the sampling device (1) further comprises a sample discharging device (15) with a discharge channel (16) and a calibration channel (17), said calibration channel (17) having a volume X which equals the volume of the measuring container (10), wherein said sample discharging device (15) is is configured to be slidably arranged to cooperate with an outlet opening (11) of the measuring container (10), and wherein the sample discharging device (15) is configured to be slidably arranged between:
(I) a closed position: wherein the outlet opening (11) of the measuring container is closed; or
(II) a discharging position: wherein the discharging channel (16) cooperates with the measuring container (10); or
(III) a calibration position: wherein the calibration channel (17) cooperates with the measuring container (10).

2. The sampling device (1) according to claim 1, wherein the sample diversion device (15) further comprises a sample diversion channel (9) with a breaking device (12) for distributing the moving flow (3) of the free flowing particulate material (2) through the sample diversion channel (9) into the measuring container (10).

3. The sampling device (1) according to anyone of claims 1-2, wherein the measuring device (14) is designed to record parameters from the group consisting of protein content, moisture content, fall number, temperature, density, water activity and the presence of molds and mycotoxins in the free flowing particulate material (2).

4. The sampling device (1) according to claim 3, wherein the measuring device (14) is designed to measure moisture content in the free flowing particulate material (2).

5. The sampling device (1) according to claim 3, wherein the measuring device (14) is designed to measure protein content in the free flowing particulate material (2).

6. The sampling device (1) according to claim 1, wherein the calibration channel (17) has an outlet opening arranged to cooperate with a collection container (21).

7. The sampling device (1) according to anyone of claims 1-6, wherein the free flowing particulate material (2) is one of the group consisting of kernels and seeds from leguminous plants cruciferous plants, cereals grass and rice.

8. The sampling device (1) according to claim 7, wherein the free flowing particulate material (2) is one of the group consisting of barley, wheat, corn, rye, brown rice, oats, rape seeds, sunflower seeds, malt, durra, peas, soya beans, peanuts, shelled peanuts, corn kernels, cotton seed, white rice and/or grass.

9. The sampling device (1) according to claim 7, wherein the free flowing particulate material (2) is kernels or seeds from cereals.

10. A method for sampling and analysis of samples from a moving flow (3) of free flowing particulate material, using the sampling device (1) according to claims 1-9, and calibration of said sampling device (1), wherein said method comprises the following steps:
a) a subset of free flowing particulate material (2) is diverted by means of the sampling diversion device (5) from the moving flow (3) of free flowing particulate material to the measuring container (10) having the volume X; and
b) the sample discharging device (15) is slidably arranged to a closed position (I), whereby the measuring container (10) is filled with a sample Y of free flowing particulate material having the volume X, and the measuring device (14) records one or more of the chosen sample parameters in said sample Y; whereafter
c) the sample discharge device is slidably arranged either
(c-1) to the discharge position (II): whereby said sample Y having the volume X is transferred from the measuring container (10) to the discharge channel (16) and is discharged from the sampling device; or
(c-2) to the calibration position (III): whereby said sample Y having the volume X is transferred from the measuring container (10) to the calibration channel (17) having a volume X which equals the volume of the measuring container (10), and thereafter to a collection container (21) for external assessment of said one or more chosen sample parameter; whereafter
(d) (a) - (d) is repeated.

11. The method according to claim 10, wherein (a) the subset of free flowing particulate material (2) is continuously diverted from the moving flow (3) of free flowing particulate material.

12. The method according to anyone of claims 10 and 11, wherein (b) a breaking device (12) distributes the free flowing particulate material (2) in the measuring container (10).

13. The method according to anyone of claims 10 - 12, wherein (b) the measuring device (14) records one or more sample parameter from the group consisting of protein content, moisture content, fall number, temperature, density, water activity and/or the presence of molds and mycotoxins in said sample Y with the volume X.

14. The method according to anyone of claims 10 - 13, wherein (c-1) said sample Y having the volume X is transferred from the measuring container (10) to the discharge channel (16) and is restored to the moving flow (3) of free flowing particulate material.

15. The method according to anyone of claims 10 - 12, wherein (c-2) after said sample Y having the volume X has been transferred to the collection container (21), the method may further comprise the following steps:
(i) the same one or more chosen sample parameters recorded in (b), are assessed by means of an external measuring device in said sample Y having the volume X, and
(ii) the value of the one or more chosen sample parameters obtained by means of the external measuring device in (i) is compared to the value of the one or more chosen sample parameters obtained by means of the measuring device (14) in (b); and
(iii) if the values for the one or more of the chosen sample parameters obtained by means of the external measuring device in (i) and the measuring device (14) in (b) respectively are the same or close, (a), (b), (c-1) and (d) are repeated, but if the values are not the same or close the measuring device (14) is readjusted and (a), (b), (c-2) and (d) are repeated.

## Patentansprüche

1. Probenentnahmevorrichtung (1) zur Probenentnahme und Analyse von freifließendem teilchenförmigem Material (2) in einem sich bewegenden Strom (3) des Materials, umfassend eine Probenumleitungsvorrichtung (5) zum Umleiten eines Teils des freifließenden teilchenförmigen Materials (2) von dem sich bewegenden Strom (3) von freifließendem teilchenförmigem Material zu einem Messbehälter (10), das ein Volumen X aufweist, und eine Messvorrichtung (14), die in Verbindung mit dem Messbehälter (10) angeordnet ist, um einen ausgewählten Probenparameter für das freifließende teilchenförmige Material (2) zu registrieren; wobei die Probenentnahmevorrichtung (1) weiter eine Probenaustragvorrichtung (15) mit einem Austragkanal (16) und einem Kalibrierkanal (17) umfasst, wobei der Kalibrierkanal (17) ein Volumen X aufweist, das gleich dem Volumen des Messbehälters (10) ist, wobei die Probenaustragvorrichtung (15) dazu ausgelegt ist, verschiebbar angeordnet zu sein, um mit einer Auslassöffnung (11) des Messbehälters (10) zusammenzuwirken, und wobei die Probenaustragvorrichtung (15) dazu ausgelegt ist, verschiebbar angeordnet zu sein zwischen:
(I) einer geschlossenen Position: wobei die Auslassöffnung (11) des Messbehälters geschlossen ist; oder
(II) einer Austragposition: wobei der Austragkanal (16) mit dem Messbehälter (10) zusammenwirkt; oder
(III) einer Kalibrierposition: wobei der Kalibrierkanal (17) mit dem Messbehälter (10) zusammenwirkt.

2. Probenentnahmevorrichtung (1) nach Anspruch 1, wobei die Probenumleitungsvorrichtung (15) weiter einen Probenumleitungskanal (9) mit einer Brechvorrichtung (12) zum Verteilen des sich bewegenden Stroms (3) des freifließenden teilchenförmigen Materials (2) durch den Probenumleitungskanal (9) in den Messbehälter (10) umfasst.

3. Probenentnahmevorrichtung (1) nach einem der Ansprüche 1-2, wobei die Messvorrichtung (14) dazu ausgelegt ist, Parameter aus der Gruppe bestehend aus Proteingehalt, Feuchtigkeitsgehalt, Fallzahl, Temperatur, Dichte, Wasseraktivität und dem Vorhandensein von Schimmelpilzen und Mykotoxinen in dem freifließenden teilchenförmigen Material (2) zu registrieren.

4. Probenentnahmevorrichtung (1) nach Anspruch 3, wobei die Messvorrichtung (14) dazu ausgelegt ist, den Feuchtigkeitsgehalt in dem freifließenden teilchenförmigen Material (2) zu messen.

5. Probenentnahmevorrichtung (1) nach Anspruch 3, wobei die Messvorrichtung (14) dazu ausgelegt ist, den Proteingehalt in dem freifließenden teilchenförmigen Material (2) zu messen.

6. Probenentnahmevorrichtung (1) nach Anspruch 1, wobei der Kalibrierkanal (17) eine Auslassöffnung aufweist, die angeordnet ist, um mit einem Sammelbehälter (21) zusammenzuwirken.

7. Probenentnahmevorrichtung (1) nach einem der Ansprüche 1-6, wobei das freifließende teilchenförmige Material (2) eines aus der Gruppe bestehend aus Kernen und Samen von Hülsenfrüchten kreuzblütigen Pflanzen, Getreiden Gras und Reis ist.

8. Probenentnahmevorrichtung (1) nach Anspruch 7, wobei das freifließende teilchenförmige Material (2) eines aus der Gruppe bestehend aus Gerste, Weizen, Mais, Roggen, Vollkornreis, Hafer, Rapssamen, Sonnenblumenkernen, Malz, Mohrenhirse, Erbsen, Sojabohnen, Erdnüssen, geschälten Erdnüssen, Maiskörnern, Baumwollsamen, weißem Reis und/oder Gras ist.

9. Probenentnahmevorrichtung (1) nach Anspruch 7, wobei das freifließende teilchenförmige Material (2) Kerne oder Samen von Getreiden sind.

10. Verfahren zur Probenentnahme und Analyse von Proben aus einem sich bewegenden Strom (3) von freifließendem teilchenförmigem Material unter Verwendung einer Probenentnahmevorrichtung (1) nach Anspruch 1-9 und Kalibrierung der Probenentnahmevorrichtung (1), wobei das Verfahren die folgenden Schritte umfasst:
a) ein Teilsatz von freifließendem teilchenförmigem Material (2) wird mittels der Probenentnahmeumleitungsvorrichtung (5) von dem sich bewegenden Strom (3) von freifließendem teilchenförmigem Material zu dem Messbehälter (10), der das Volumen X aufweist, umgeleitet; und
b) die Probenaustragvorrichtung (15) wird verschiebbar in eine geschlossene Position (I) angeordnet, wobei der Messbehälter (10) mit einer Probe Y von freifließendem teilchenförmigem Material, das das Volumen X aufweist, gefüllt wird und die Messvorrichtung (14) einen oder mehrere der ausgewählten Probenparameter in der Probe Y registriert; wonach
c) die Probenaustragvorrichtung verschiebbar angeordnet wird entweder
(c-1) in die Austragposition (II): wobei die Probe Y, die das Volumen X aufweist, von dem Messbehälter (10) in den Austragkanal (16) übertragen wird und aus der Probenentnahmevorrichtung ausgetragen wird; oder
(c-2) in die Kalibrierposition (III): wobei die Probe Y, die das Volumen X aufweist, von dem Messbehälter (10) in den Kalibrierkanal (17), der ein Volumen X aufweist, das gleich dem Volumen des Messbehälters (10) ist, und danach in einen Sammelbehälter (21) zur externen Bewertung des einen oder der mehreren ausgewählten Probenparameter übertragen wird; wonach
(d) (a)-(d) wiederholt wird.

11. Verfahren nach Anspruch 10, wobei (a) der Teilsatz von freifließendem teilchenförmigem Material (2) fortlaufend von dem sich bewegenden Strom (3) von freifließendem teilchenförmigem Material umgeleitet wird.

12. Verfahren nach einem der Ansprüche 10 und 11, wobei (b) eine Brechvorrichtung (12) das freifließende teilchenförmige Material (2) in dem Messbehälter (10) verteilt.

13. Verfahren nach einem der Ansprüche 10 - 12, wobei (b) die Messvorrichtung (14) einen oder mehrere Probenparameter aus der Gruppe bestehend aus Proteingehalt, Feuchtigkeitsgehalt, Fallzahl, Temperatur, Dichte, Wasseraktivität und/oder dem Vorhandensein von Schimmelpilzen und Mykotoxinen in der Probe Y mit dem Volumen X registriert.

14. Verfahren nach einem der Ansprüche 10 - 13, wobei (c-1) die Probe Y, die das Volumen X aufweist, von dem Messbehälter (10) zu dem Austragkanal (16) übertragen wird und in den sich bewegenden Strom (3) von freifließendem teilchenförmigem Material zurückgeführt wird.

15. Verfahren nach einem der Ansprüche 10 - 12, wobei (c-2), nachdem die Probe Y, die das Volumen X aufweist, zu dem Sammelbehälter (21) übertragen wurde, das Verfahren weiter die folgenden Schritte umfassen kann:
(i) der gleiche eine oder die gleichen mehreren ausgewählten Probenparameter, die in (b) erfasst wurden, werden mittels einer externen Messvorrichtung in der Probe Y, die das Volumen X aufweist, registriert und
(ii) der Wert des einen oder der mehreren ausgewählten Probenparameter, die mittels der externen Messvorrichtung in (i) erhalten wurden, wird mit dem Wert des einen oder der mehreren ausgewählten Probenparameter, die mittels der Messvorrichtung (14) in (b) erhalten wurden, verglichen; und
(iii) wenn die Werte für den einen oder die mehreren der ausgewählten Probenparameter, die mittels der externen Messvorrichtung in (i) bzw. der Messvorrichtung (14) in (b) erhalten wurden, gleich sind oder nahe beieinander liegen, werden (a), (b), (c-1) und (d) wiederholt, aber wenn die Werte nicht gleich sind oder nahe beieinander liegen, wird die Messvorrichtung (14) neu eingestellt und (a), (b), (c-2) und (d) werden wiederholt.

## Revendications

1. Dispositif d'échantillonnage (1) pour l'échantillonnage et l'analyse de matière particulaire à écoulement libre (2) dans un écoulement mobile (3) de ladite matière, comprenant un dispositif de déviation d'échantillon (5) pour faire dévier une partie de la matière particulaire à écoulement libre (2) depuis l'écoulement mobile (3) de matière particulaire à écoulement libre vers un récipient de mesure (10) ayant un volume X, et un dispositif de mesure (14) agencé en raccordement avec le récipient de mesure (10) pour enregistrer un paramètre d'échantillon choisi pour la matière particulaire à écoulement libre (2) ; dans lequel le dispositif d'échantillonnage (1) comprend en outre un dispositif de décharge d'échantillon (15) avec un canal de décharge (16) et un canal d'étalonnage (17), ledit canal d'étalonnage (17) ayant un volume X qui est égal au volume du récipient de mesure (10), dans lequel ledit dispositif de décharge d'échantillon (15) est configuré pour être agencé de manière coulissante afin de coopérer avec une ouverture de sortie (11) du récipient de mesure (10), et dans lequel le dispositif de décharge d'échantillon (15) est configuré pour être agencé de manière coulissante entre :
(I) une position fermée : dans laquelle l'ouverture de sortie (11) du récipient de mesure est fermée ; ou
(II) une position de décharge : dans laquelle le canal de décharge (16) coopère avec le récipient de mesure (10) ; ou
(III) une position d'étalonnage : dans laquelle le canal d'étalonnage (17) coopère avec le récipient de mesure (10).

2. Dispositif d'échantillonnage (1) selon la revendication 1, dans lequel le dispositif de déviation d'échantillon (15) comprend en outre un canal de déviation d'échantillon (9) avec un dispositif de rupture (12) pour distribuer l'écoulement mobile (3) de la matière particulaire à écoulement libre (2) à travers le canal de déviation d'échantillon (9) dans le récipient de mesure (10).

3. Dispositif d'échantillonnage (1) selon l'une quelconque des revendications 1-2, dans lequel le dispositif de mesure (14) est conçu pour enregistrer des paramètres à partir du groupe constitué par la teneur en protéines, la teneur en humidité, le nombre de chutes, la température, la densité, l'activité de l'eau et la présence de moisissures et de mycotoxines dans la matière particulaire à écoulement libre (2).

4. Dispositif d'échantillonnage (1) selon la revendication 3, dans lequel le dispositif de mesure (14) est conçu pour mesurer la teneur en humidité dans la matière particulaire à écoulement libre (2).

5. Dispositif d'échantillonnage (1) selon la revendication 3, dans lequel le dispositif de mesure (14) est conçu pour mesurer la teneur en protéines dans la matière particulaire à écoulement libre (2).

6. Dispositif d'échantillonnage (1) selon la revendication 1, dans lequel le canal d'étalonnage (17) comporte une ouverture de sortie agencée pour coopérer avec un récipient de collection (21).

7. Dispositif d'échantillonnage (1) selon l'une quelconque des revendications 1-6, dans lequel la matière particulaire à écoulement libre (2) est l'une du groupe constitué par des grains et des graines provenant de plantes légumineuses, de plantes crucifères, des céréales, de l'herbe et du riz.

8. Dispositif d'échantillonnage (1) selon la revendication 7, dans lequel la matière particulaire à écoulement libre (2) est l'un du groupe constitué par l'orge, le blé, le maïs, le seigle, le riz brun, l'avoine, les graines de colza, les graines de tournesol, le malt, la doura, les pois, les graines de soja, les arachides, les arachides écalées, les grains de maïs, les graines de coton, le riz blanc et/ou l'herbe.

9. Dispositif d'échantillonnage (1) selon la revendication 7, dans lequel la matière particulaire à écoulement libre (2) est des grains ou des graines provenant de céréales.

10. Procédé pour l'échantillonnage et l'analyse d'échantillons provenant d'un écoulement mobile (3) de matière particulaire à écoulement libre, en utilisant le dispositif d'échantillonnage (1) selon les revendications 1-9, et l'étalonnage dudit dispositif d'échantillonnage (1), dans lequel ledit procédé comprend les étapes suivantes :
a) un sous-ensemble de matière particulaire à écoulement libre (2) est dévié à l'aide du dispositif de déviation d'échantillon (5) depuis l'écoulement mobile (3) de matière particulaire à écoulement libre vers le récipient de mesure (10) ayant le volume X ; et
b) le dispositif de décharge d'échantillon (15) est agencé de manière coulissante jusqu'à une position fermée (I), selon laquelle le récipient de mesure (10) est rempli d'un échantillon Y de matière particulaire à écoulement libre ayant le volume X, et le dispositif de mesure (14) enregistre un ou plusieurs des paramètres d'échantillon choisis dans ledit échantillon Y ; après quoi
c) le dispositif de décharge d'échantillon est agencé de manière coulissante soit
(c-1) vers la position de décharge (II) : selon laquelle ledit échantillon Y ayant le volume X est transféré depuis le récipient de mesure (10) vers le canal de décharge (16) et est déchargé du dispositif d'échantillonnage ; soit
(c-2) vers la position d'étalonnage (III) : selon laquelle ledit échantillon Y ayant le volume X est transféré depuis le récipient de mesure (10) vers le canal d'étalonnage (17) ayant un volume X qui est égal au volume du récipient de mesure (10), et ensuite vers un récipient de collecte (21) pour l'évaluation externe desdits un ou plusieurs paramètres d'échantillon choisis ; après quoi
(d) (a)-(d) sont répétées.

11. Procédé selon la revendication 10, dans lequel (a) le sous-ensemble de matière particulaire à écoulement libre (2) est dévié de façon continue depuis l'écoulement mobile (3) de matière particulaire à écoulement libre.

12. Procédé selon l'une quelconque des revendications 10 et 11, dans lequel (b) un dispositif de rupture (12) distribue la matière particulaire à écoulement libre (2) dans le récipient de mesure (10).

13. Procédé selon l'une quelconque des revendications 10-12, dans lequel (b) le dispositif de mesure (14) enregistre un ou plusieurs paramètres d'échantillon à partir du groupe constitué par la teneur en protéines, la teneur en humidité, le nombre de chutes, la température, la densité, l'activité de l'eau et/ou la présence de moisissures et de mycotoxines dans ledit échantillon Y avec le volume X.

14. Procédé selon l'une quelconque des revendications 10-13, dans lequel (c-1) ledit échantillon Y ayant le volume X est transféré depuis le récipient de mesure (10) vers le canal de décharge (16) et est ramené à l'écoulement mobile (3) de matière particulaire à écoulement libre.

15. Procédé selon l'une quelconque des revendications 10-12, dans lequel (c-2) après le transfert dudit échantillon Y ayant le volume X vers le récipient de collecte (21), le procédé peut en outre comprendre les étapes suivantes :
(i) les mêmes un ou plusieurs paramètres d'échantillon choisis enregistrés en (b) sont évalués à l'aide d'un dispositif de mesure externe dans ledit échantillon Y ayant le volume X, et
(ii) la valeur des un ou plusieurs paramètres d'échantillon choisis obtenue à l'aide du dispositif de mesure externe en (i) est comparée à la valeur des un ou plusieurs paramètres d'échantillon choisis obtenue à l'aide du dispositif de mesure (14) en (b) ; et
(iii) si les valeurs pour les un ou plusieurs des paramètres d'échantillon choisis obtenues à l'aide du dispositif de mesure externe en (i) et du dispositif de mesure (14) en (b) respectivement sont les mêmes ou proches, (a), (b), (c-1) et (d) sont répétées, mais si les valeurs ne sont pas les mêmes ou proches le dispositif de mesure (14) est réajusté et (a), (b), (c-2) et (d) sont répétées.
